# EUROPÄISCHE PATENTANMELDUNG

(11) **EP 3 069 757 A1**
(43) Veröffentlichungstag der Anmeldung: **21.09.2016**
(21) Anmeldenummer: 16157065.0
(22) Anmeldetag: 24.02.2016
(51) Int. Cl.: A61N 1/375

(54) **DURCHFÜHRUNG EINES IMPLANTIERBAREN MEDIZINELEKTRONISCHEN GERÄTES UND IMPLANTIERBARES MEDIZINELEKTRONISCHES GERÄT**

(30) Priorität: 20.03.2015 US 201562135709 P
(71) Anmelder: BIOTRONIK SE & Co. KG, 12359 Berlin (DE)
(72) Erfinder: Starke, Marcel, 15732 Eichwalde (DE)
(74) Vertreter: Galander, Marcus

(57) **Zusammenfassung**

Durchführung eines implantierbaren medizinelektronischen Geräts, mit einem Gehäuse, einem Isolierkörper, einem den Isolierkörper umfassenden Durchführungs-Flansch und mindestens einem den Isolierkörper durchstoßenden Anschlusselement zum externen Anschluss eines elektrischen oder elektronischen Bauelements des Geräts, insbesondere mehreren Anschlusselementen, wobei zwischen dem Isolierkörper und dem Durchführungs-Flansch und/oder zwischen dem Isolierkörper und dem oder mindestens einem Anschlusselement und/oder zwischen dem Isolierkörper und dem Gehäuse eine Niedertemperatur-Hartlötverbindung oder Weichlötverbindung vorgesehen ist, die insbesondere bei einer Temperatur von 900°C oder weniger, bevorzugt weniger als 450°C, noch bevorzugter weniger als 400°C, gebildet ist.

## Beschreibung

Die Erfindung betrifft eine Durchführung eines implantierbaren medizinelektronischen Geräts, mit einem Isolierkörper, einem den Isolierkörper umfassenden Durchführungs-Flansch und mindestens einem den Isolierkörper durchstoßenden Anschlusselement zum externen Anschluss eines elektrischen oder elektronischen Bauelements des Geräts, insbesondere mehreren Anschlusselementen. Sie betrifft des Weiteren ein implantierbares medizinielektronisches Gerät, welches eine solche Durchführung enthält.

Derartige Geräte sind insbesondere als Herzschrittmacher oder implantierbare Kardioverter (speziell Defibrillatoren) seit langem in massenhaftem Einsatz. Es kann sich hierbei aber auch um eine weniger komplexe Vorrichtung, wie etwa eine Elektroden- oder Sensorleitung oder auch ein Cochlea-Implantat, handeln.

Die meisten praktisch bedeutsamen implantierbaren elektromedizinischen Geräte sind dazu vorgesehen, über geeignet platzierte Elektroden elektrische Impulse an reizbares Körpergewebe abzugeben. Um diese Funktion auszuführen, sind im Gehäuse des Gerätes elektronische/elektrische Funktionseinheiten zur Erzeugung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet.

Bekannt sind insbesondere Durchführungen, deren Grund- und Isolationskörper im Wesentlichen aus Keramik oder Glas besteht, wobei auch mehrschichtige bzw. mehrteilige Aufbauten unter Einsatz von Metallen oder Metalloxiden entwickelt wurden und eingesetzt werden. Derartige bekannte Durchführungen erfüllen weitgehend die an sie gestellten Anforderungen. Jedoch müssen bei der Materialauswahl für die Komponenten Isolationskeramik/Glas, Metall- oder Glaslot, Metallpin und Metallflansch die thermischen Ausdehnungskoeffizienten berücksichtigt werden, um eine über die vorgesehene Lebensdauer ausreichende Dichtheit gewährleisten zu können.

Beim konventionellen Design (Metallflansch - Lot - Isolationskeramik - Lot - Metallpin) kommt die Wirkung von nicht angepassten thermischen Ausdehnungskoeffizienten in erster Linie beim Abkühlen von Löttemperatur und Einschweißen der Durchführung ins Gehäuse zum Tragen. Resultieren können mechanische Zugspannungen, welche zur Materialtrennung und folglich zu etwaigen Leckagen der Durchführung führen können. Die bei konventionellen Durchführungen verwendeten keramischen und metallischen Komponenten sind durch den Lotwerkstoff miteinander verbunden; bei ungleichmäßiger Ausdehnung/Schrumpfung der Komponenten untereinander, einschließlich des Lotes, aufgrund von Aufheiz-/Abkühlvorgängen erzeugen die resultierenden relativen Längenänderungen entsprechende mechanische Spannungen.

Aus der EP 2 232 646 B1 ist eine hermetisch dichte Durchführungs-Struktur bekannt, die einen mehrteiligen Grund- bzw. Isolationskörper in Kombination mit abdichtenden (nicht strukturtragenden) Polymerschichten umfasst. Die Herstellung einer solchen Durchführung ist höchst aufwändig hinsichtlich der erforderlichen Arbeits- und Prüfschritte und auch hinsichtlich der Vorfertigung, Lagerhaltung und Zuführung vieler verschiedener Teile.

Auch die US 7,064,270 B2 beschreibt eine mehrteilig aufgebaute Durchführung, die speziell für eine Elektrodenleitung entwickelt wurde und mehrere aus Kunststoff gefertigte oder mit einer Kunststoffbeschichtung versehene Komponenten umfassen kann.

Aus der EP 2 388 044 A1 ist ein elektronisches Gerät bekannt, welches eine grundsätzlich einfach aufgebaute Durchführung aus einem flüssigkristallinen Polymeren hat. Einzelheiten der Gerätekonstruktion werden in dieser Druckschrift nicht offenbart.

Es ist auch bekannt, Hartlötverfahren zur stoffschlüssigen Verbindung etwa von Titan- und Nickel-Teilen unter Einsatz eutektischer Lote durchzuführen und hierdurch die mit Schweißverbindungen oder hochtemperatur-Lötverfahren einhergehenden thermischen Belastungen und daraus resultierenden Probleme zu verringern; vgl. N. Weyrich et. al. "Joining of Titanium and Nickel at Temperatures Below 450°C", Brazing, High Temperature Brazing and Diffusion Bonding, Löt 2013, p. 22. Bekannt ist auch der Einsatz niedrig schmelzender Au-Legierungs-Lote für das Einlöten von Filterkondensatoren in Durchführungen implantierbarer medizinischer Geräte; vgl. dazu US 5,870,272 oder US 6,031,710.

Der Erfindung liegt die Aufgabe zu Grunde, ein verbessertes implantierbares elektromedizinisches Gerät bereitzustellen, welches kostengünstig herstellbar und hochgradig zuverlässig ist.

Diese Aufgabe wird durch eine Durchführung mit den Merkmalen des Anspruchs 1 gelöst. Zweckmäßige Fortbildungen des Erfindungsgedankens sind Gegenstand der abhängigen Ansprüche. Des Weiteren wird ein entsprechendes implantierbares medizinelektronisches Gerät vorgeschlagen.

Die Erfindung geht von der Überlegung aus, die für eine dauerhaft zuverlässige Funktion der Durchführung grundsätzlich kritischen Aufheiz- und Abkühlschritte mit deutlich geringeren Temperaturdifferenzen auszuführen und hierdurch die erwähnten Probleme zumindest wesentlich zu verringern. Hinzu tritt die Überlegung, dass verringerte maximale Prozesstemperaturen Potential für den Einsatz weniger temperaturbeständiger Materialien und generell größere Freiheitsgrade beim Entwurf der Durchführung bieten. Dies mündet in der Überlegung, zwischen dem Isolierkörper und dem Durchführungs-Flansch und/oder zwischen dem Isolierkörper und dem oder mindestens einem Anschlusselement und/oder zwischen dem Isolierkörper und dem Gehäuse eine Niedertemperatur-Hartlötverbindung oder Weichlötverbindung vorzusehen, die insbesondere bei einer Temperatur von 900°C oder weniger, bevorzugt weniger als 450°C, noch bevorzugter weniger als 400°C, gebildet ist.

In einer Ausführung der Erfindung umfasst die Durchführung die Niedertemperatur-Hartlötverbindung mit einem unterhalb von 900°C, insbesondere unterhalb von 450°C und weiter insbesondere unterhalb von 400°C, schmelzenden eutektischen Gold-Legierungs-Lot gebildet ist, wie Au80Sn20, Au81Si19, Au94Sn6 oder Ähnlichem.

In weiteren Ausführungen der Erfindung ist vorgesehen, dass die Durchführung die einen Bestandteil oder Bereich umfasst, der nur bis zu einer Temperatur von 1050°C, insbesondere nur bis 950°C oder weniger, begrenzt temperaturstabil ist.

In einer Ausgestaltung dieser Ausführungen ist vorgesehen, dass der begrenzt temperaturstabile Bestandteil oder Bereich einen Metall/Keramik-Verbund aufweist.

In einer Variante dieser Ausführung weist der begrenzt temperaturstabile Bestandteil eine in LTCC-Technologie erstellte elektronische Anordnung, insbesondere Anordnung von Anschlusselementen, auf.

In weiteren Ausführungen weist der Isolierkörper mindestens eine Kunststoffkomponente auf. In einer Ausgestaltung ist vorgesehen, dass die Kunststoffkomponente ein Kunststoff Spritzgussteil oder eine Kunststoffbeschichtung eines Keramik- oder Glasteiles ist. Des Weiteren kann die Kunststoffkomponente eine Füllung mit nichtorganischen und nichtmetallischen Teilchen, insbesondere Keramik- und/oder Glasteilchen aufweisen.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische, teilweise geschnittene Darstellung eines implantierbaren elektromedizinischen Geräts,
- Fig. 2: eine schematische Querschnittsdarstellung (Teilansicht) eines Ausführungsbeispiels der Erfindung,
- Fig. 3: eine schematische Querschnittsdarstellung (Teilansicht) eines weiteren Ausführungsbeispiels der Erfindung, und
- Fig. 4: eine schematische Querschnittsdarstellung (Teilansicht) eines weiteren Ausführungsbeispiels der Erfindung.

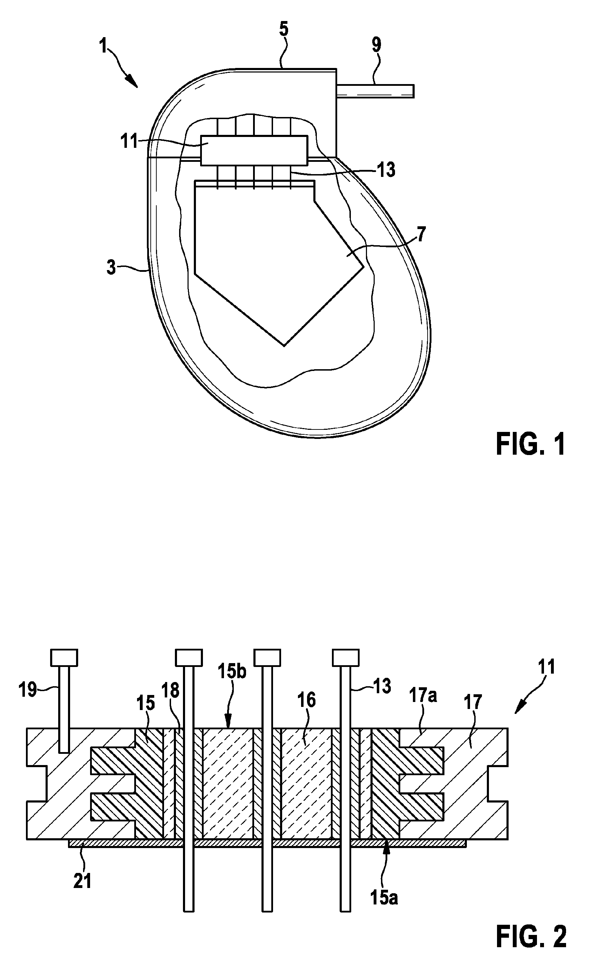

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser weich verlötet.

Fig. 2 zeigt, unter Verwendung der gleichen Bezugsziffern für funktionsgleiche Teile wie in Fig. 1, beispielhaft einen Aufbau einer Durchführung 11. Diese umfasst einen als Spritzgussteil erzeugten äußeren, ringförmigen Kunststoff-Grundkörper 15 und inneren, scheibenförmigen Keramik-Grundkörper 16, eingesetzt in einen durch Metall-Pulverspritzgießen (MiM-Technologie) gebildeten Durchführungs-Flansch 17. Der Flansch 17 trägt an seinem Innenumfang mehrere nach innen in das Material des in den Flansch direkt eingespritzten Kunststoff-Grundkörpers 15 ragende ringförmige Fortsätze 17a. Diese sorgen für eine mehrfache Verzahnung mit dem Kunststoffmaterial und somit für eine hermetisch dichte Verbindung zwischen dem äußeren Kunststoff-Grundkörper 15 und dem Flansch 17. Der Außenumfang des inneren Keramik-Grundkörpers 16 weist in der figürlichen Darstellung keine derartigen Fortsätze auf, in der Praxis können solche aber auch dort vorgesehen sein und eine vergleichbare Wirkung entfalten wie beim Flansch 17.

Mehrere Anschlussstifte 13 durchstoßen den inneren Keramik-Grundkörper 16. Sie sind in diesen jeweils mit einer Weichlötverbindung 18 aus einem eutektischen Gold-Legierungs-Lot stoffschlüssig eingefügt. Durch den Einsatz der niedertemperatur-Lötverbindung wird das Einlöten der Anschlussstifte 13 in den inneren Keramik-Grundkörper 16 ungeachtet dessen möglich, dass der umgebende äußere Kunststoff-Grundkörper 15 eine begrenzte Temperaturbeständigkeit hat und den bei einem herkömmlichen Hartlötverfahren benötigten Temperaturen nicht widerstehen würde.

In den Flansch 17 ist im Übrigen ein Massepin 19 eingefügt (wiederum eingelötet oder im MiM-Verfahren zugleich erzeugt). Eine beide Grundkörper 15, 16 auf der DurchführungOberfläche 15a bedeckende Barriereschicht 21 verbessert die Diffusionsfestigkeit der Durchführung gegenüber gasförmigen oder flüssigen Bestandteilen der Einsatzumgebung des Gerätes.

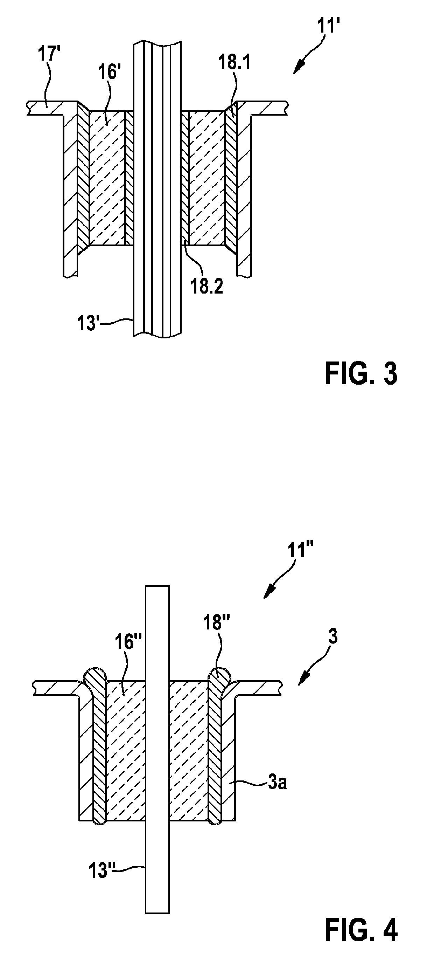

Fig. 3 stellt in einer sehr vereinfachten schematischen Darstellung eine weitere Ausführung einer Durchführung 11' dar, bei der ein Keramik-Isolierkörper 16' sowohl an seiner Außenwandung über eine Niedertemperatur-Hartlötverbindung 18.1 mit einem kalt umgeformten Durchführungs-Flansch 17' verbunden als auch im Inneren des Grundkörpers eine Niedertemperatur-Hartlötverbindung 18.2 zum hermetisch dichten Einbetten einer in LTCC-Technologie erstellten, relativ hitzeempfindlichen Anschlusselement-Anordnung 13' vorgesehen ist. Unter einer Niedertemperatur-Hartlötverbindung wird hier eine solche verstanden, die in freier Atmosphäre, d.h. nicht im Vakuum oder unter Schutzgas, erzeugt werden kann. Auch hier ermöglicht die Anwendung eines eutektischen Niedertemperatur-Lotes nicht nur die Reduzierung von Spannungsbelastungen und eine Erhöhung der generellen Zuverlässigkeit, sondern vor Allem auch ein neuartiges Konstruktionsprinzip der Durchführung.

Fig. 4 stellt, wiederum in einer sehr schematisierten Darstellung, eine weitere beispielhafte Durchführung 11" dar, bei der ein Keramik-Isolierkörper 16", der hier einen einzelnen Anschlussstift 13" umgibt, mittels einer Niedertemperatur-Hartlötverbindung 18" direkt mit einem umgebogenen Abschnitt 3a eines Implantatgehäuses 3 verlötet ist.

Die Ausführung der Erfindung ist auch in einer Vielzahl von Abwandlungen der hier gezeigten Beispiele und weiter oben hervorgehobenen Aspekte der Erfindung möglich.

## Patentansprüche

1. Durchführung (11; 11') eines implantierbaren medizinelektronischen Geräts (1), mit einem Gehäuse, einem Isolierkörper (15, 16; 16'), einem den Isolierkörper umfassenden Durchführungs-Flansch (17, 17') und mindestens einem den Isolierkörper durchstoßenden Anschlusselement (13; 13') zum externen Anschluss eines elektrischen oder elektronischen Bauelements des Geräts, insbesondere mehreren Anschlusselementen,
wobei zwischen dem Isolierkörper und dem Durchführungs-Flansch und/oder zwischen dem Isolierkörper und dem oder mindestens einem Anschlusselement und/oder zwischen dem Isolierkörper und dem Gehäuse eine Niedertemperatur-Hartlötverbindung oder Weichlötverbindung (18; 18.1, 18.2) vorgesehen ist, die insbesondere bei einer Temperatur von 900°C oder weniger, bevorzugt weniger als 450°C, noch bevorzugter weniger als 400°C, gebildet ist.

2. Durchführung nach Anspruch 1, wobei die Niedertemperatur-Hartlötverbindung (18; 18.1, 18.2) mit einem unterhalb von 900°C, insbesondere unterhalb von 450°C und weiter insbesondere unterhalb von 400°C, schmelzenden eutektischen Gold-Legierungs-Lot gebildet ist, wie Au80Sn20, Au81Si19, Au94Sn6 oder Ähnlichem.

3. Durchführung nach Anspruch 1, die einen Bestandteil oder Bereich (15; 13') umfasst, der nur bis zu einer Temperatur von 1050°C, insbesondere nur bis 950°C oder weniger, begrenzt temperaturstabil ist.

4. Durchführung nach Anspruch 3, wobei der begrenzt temperaturstabile Bestandteil oder Bereich ein Metall/Keramik-Verbund aufweist.

5. Durchführung nach Anspruch 3 oder 4, wobei der begrenzt temperaturstabile Bestandteil eine in LTCC-Technologie erstellte elektronische Anordnung (13'), insbesondere Anordnung von Anschlusselementen, aufweist.

6. Durchführung nach Anspruch 3, wobei der Isolierkörper mindestens eine Kunststoffkomponente (15) aufweist.

7. Durchführung nach Anspruch 6, wobei die Kunststoffkomponente (15) ein Kunststoff-Spritzgussteil oder eine Kunststoffbeschichtung eines Keramik- oder Glasteiles ist.

8. Durchführung nach Anspruch 6 oder 7, wobei die Kunststoffkomponente (15) eine Füllung mit nichtorganischen und nichtmetallischen Teilchen, insbesondere Keramik- und/oder Glasteilchen aufweist.

9. Implantierbares medizinelektronisches Gerät (1) mit einer Durchführung (11; 11') nach einem der vorangehenden Ansprüche, insbesondere ausgeführt als Herzschrittmacher, implantierbarer Kardioverter oder Cochlear-Implantat.
